# EUROPÄISCHE PATENTSCHRIFT

(11) **EP 0 613 663 B1**
(45) Veröffentlichungstag und Bekanntmachung des Hinweises auf die Patenterteilung: **22.07.1998**
(21) Anmeldenummer: 93118420.4
(22) Anmeldetag: 15.11.1993
(51) Int. Cl.: A61B 17/72, A61B 17/74

(54) **Modularer Marknagel**
Modular intramedullary nail
Cheville intramédullaire à module

(30) Priorität: 04.12.1992 WO PCT/CH92/00235
(43) Veröffentlichungstag der Anmeldung: 07.09.1994
(73) Patentinhaber: SYNTHES AG, 7002 Chur (CH)
(72) Erfinder: Frigg, Robert, CH-7270 Davos-Platz (CH)
(74) Vertreter: Lusuardi, Werther Giovanni, Dr.

(56) Entgegenhaltungen:
- EP-A- 0 306 709
- DE-U- 9 115 200
- US-A- 4 622 959
- US-A- 4 805 607
- US-A- 5 122 141

## Beschreibung

Die Erfindung bezieht sich auf einen Marknagel gemäss dem Oberbegriff des Anspruchs 1.

Marknägel sind nagelförmige Kraftträger, mit welchen bei der sogenannten Marknagelung ein Knochenbruch durch mechanische Überbrückung intramedullär stabilisiert werden kann.

Bis vor kurzer Zeit wurden Marknägel in konventionelle Marknägel und Verriegelungsnägel unterteilt. Die Überlegungen, die in der Fachwelt zu dieser Unterscheidung führten, beruhten auf der Ansicht, dass der konventionelle Marknagel trotz fehlender Verriegelungsmöglichkeit der als Standard zu verwendende Marknagel sei. Diese Auffassung ist inzwischen überholt und es besteht ein starker Trend in Richtung des Verriegelungsnagels als Standardnagel. Doch auch innerhalb der Kategorie der Verriegelungsnägel zeichnet sich eine Tendenz zu unaufgebohrten Marknägeln sowie Marknägeln mit verschiedenartigen Verriegelungsmöglichkeiten ab. Bei den für den Femur bestimmten Marknägeln ist vor allem deren Vielfalt - mit unterschiedlicher Verriegelung - beträchtlich. Der Grund dafür liegt in der Komplexität der Femur-Frakturen und deren Behandlung. Je nach Art und Lage der Fraktur muss entsprechend die Art und die Lage der proximalen Verriegelung anders gewählt werden.

Die verschiedenartige Lage und Art der proximalen Verriegelung von Femur-Marknägeln hat zu einer verwirrenden Vielzahl von konstruktiven Abarten geführt. Dies ist als äusserst nachteilig einzustufen. Einerseits entstehen dadurch hohe Lagerkosten für die Spitäler, da alle Dimensionen und Typen vorrätig sein müssen, da die Operation unmittelbar bei der Einlieferung des Patienten erfolgen muss. Andererseits müssen eine Vielzahl von verschiedenen Instrumentarien bereitgelegt werden, was ebenfalls mit einem hohen Aufwand verbunden ist. Speziell für kleinere Kliniken, die insgesamt den Grossteil der Patienten weltweit behandeln, sind die Kosten und die Komplexität der Lagerhaltung und der Instrumentarien oft der Grund auf andere - wenn auch weniger elegante - Frakturbehandlungsmethoden auszuweichen.
Aus der DE-U-9115200 ist bereits ein Verriegelungsnagel nach dem Oberbegriff des Patentanspruchs 1 bekannt.

Hier will die Erfindung Abhilfe schaffen. Der Erfindung liegt die Aufgabe zugrunde einen Marknagel zu schaffen, welcher das Implantate- und Instrumentensortiment auf ein Minimum reduziert, die anzuwendende Operationstechnik vereinfacht und die relative Lage von Marknagel und zugehöriger Hülse eindeutig definiert.

Der erfindungsgemässe Marknagel zeichnet sich durch einen modularen Aufbau aus, der an die konkret vorliegende Knochenfraktur angepasst werden kann. Grundsätzlich lässt sich der erfindungsgemässe Marknagel für sämtliche Marknagel-Indikationen am Femur und anderen Röhrenknochen verwenden.

Die Erfindung löst die gestellte Aufgabe mit einem Marknagel, welcher die Merkmale des Anspruchs 1 aufweist. Weitere vorteilhafte Ausgestaltungen der Erfindung sind in den abhängigen Ansprüchen gekennzeichnet.

Ein Ausführungsbeispiel der Erfindung, welches zugleich das Funktionsprinzip erläutert, ist in der Zeichnung dargestellt und wird im folgenden näher beschrieben. Obwohl das Ausführungsbeispiel lediglich anhand eines Femur-Marknagels illustriert wird, kann der erfindungsgemässe Marknagel unter entsprechender Anpassung an die anatomischen Verhältnisse auch für andere Röhrenknochen verwendet werden.
Fig. 1 zeigt eine Seitenansicht des erfindungsgemässen Marknagels mit Hülse im demontierten Zustand;
Fig. 2 zeigt eine vergrösserte Seitenansicht des proximalen Abschnitts des Marknagels nach Fig. 1;
Fig. 3 zeigt eine vergrösserte Seitenansicht der Hülse nach Fig. 1;
Fig. 4 zeigt eine Aufsicht auf die Hülse nach Fig. 3;
Fig. 5 zeigt einen um 90° versetzten Längsschnitt durch den Marknagel nach Fig. 2;
Fig. 6 zeigt einen um 90° versetzten Längsschnitt durch die Hülse nach Fig. 3;
Fig. 7 zeigt den erfindungsgemässen Marknagel im zusammengesetzten Zustand mit einer nach unten geneigten Knochenschraube als Knochenfixationsmittel; und
Fig. 8 zeigt den erfindungsgemässen Marknagel im zusammengesetzten Zustand mit einer nach oben geneigten Knochenschraube als Knochenfixationsmittel.

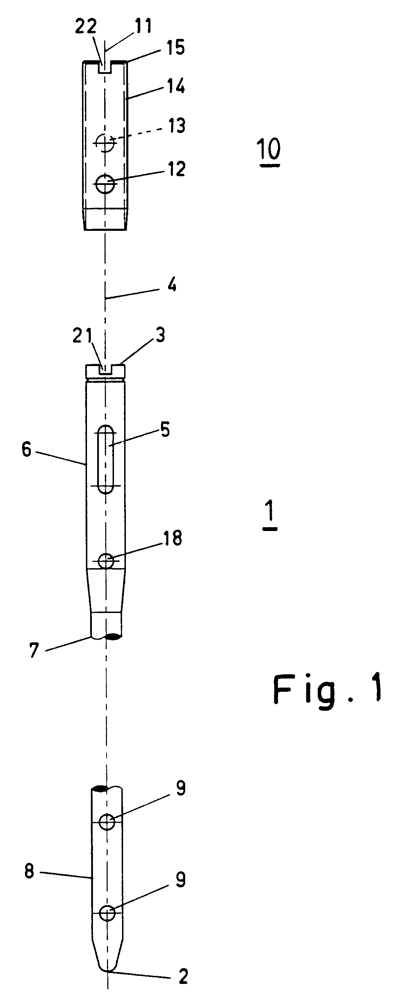

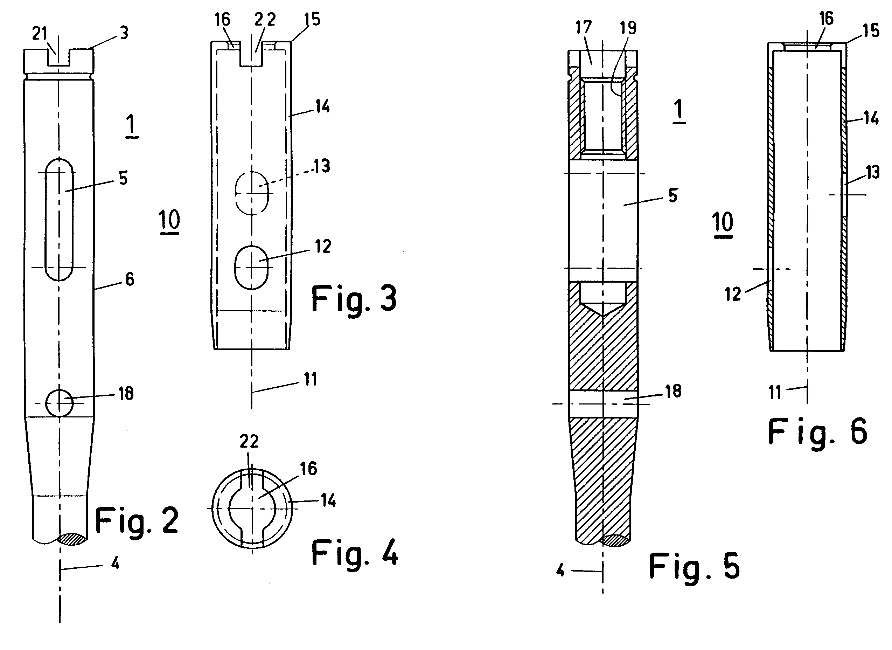

Der in den Fig. 1 - 8 dargestellte Marknagel 1, der hier als Femur-Marknagel ausgestaltet ist, besteht im wesentlichen aus einem runden Stab mit vollem Querschnitt. Der Marknagel 1 weist in Richtung seiner Längsachse 4 drei verschiedene Teilbereiche auf: Einen proximalen Abschnitt 6, einen zentralen Abschnitt 7 und einen distalen Abschnitt 8 mit der Nagelspitze 2. Die zentralen und distalen Abschnitte 7,8 sind im Durchmesser so dimensioniert, dass sie in den Markraum des Femurs geschoben werden können, ohne dass letzterer aufgebohrt werden muss. Im Bereich des distalen Abschnitts 8 befinden sich die distalen Verriegelungslöcher 9.

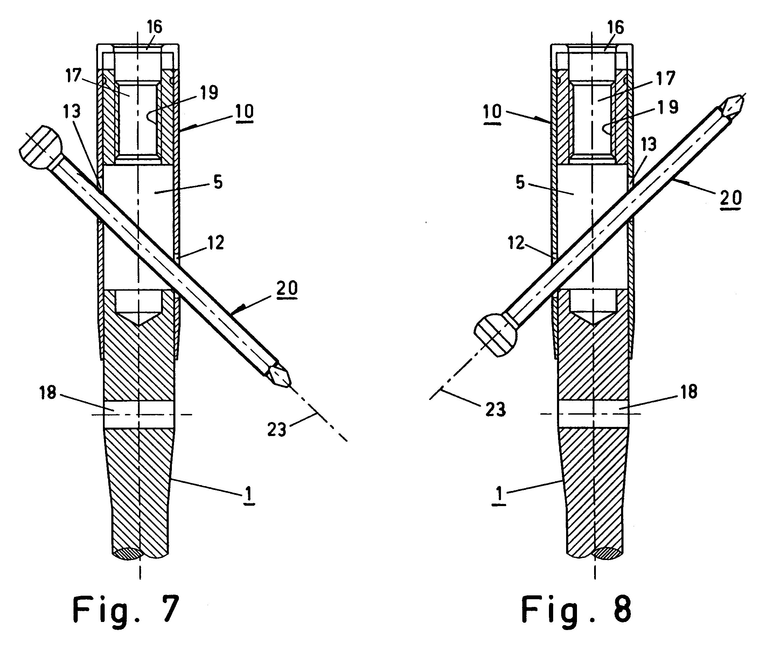

Der proximale Abschnitt 6 hat einen gegebenen Durchmesser, der unabhängig vom Durchmesser der beiden anderen Abschnitte 7,8 ist. Er weist zudem einen als Langloch 5 ausgebildeten durchgängigen Schlitz mit einer Länge von 6 - 40 mm, vorzugsweise von 20 - 30 mm auf, durch den, wie in den Fig. 7 und 8 dargestellt, verschiedenartige Verriegelungslemente 20, z.B. Knochenschrauben oder Knochenklingen geführt werden können. Eine unterhalb des Langslochs 5 angebrachte Querbohrung 18 erlaubt die Aufnahme einer in Lage und Richtung fixierten Verriegelungsschraube, welche jedoch auch weggelassen werden kann.

Die zeichnerisch nicht dargestellten, durchaus konventionellen Instrumente zur Implantation und zur Extraktion des Marknagels 1 können in einer am proximalen Nagelende 3 angebrachten zentralen Bohrung 17 mit Innengewinde 19 fixiert werden. Die quer zur Längsachse 4 verlaufende Nute 21 am proximalen Nagelende 3 dient zur Ausrichtung der erwähnten Instrumente um die als Rotationsachse gedachte Längsachse 4.

Wird der erfindungsgemässe Marknagel 1 zur Versorgung einer Mittschaftfraktur verwendet, wird er ohne Zusatz, d.h. völlig konventionell, so wie oben beschrieben, in den Markraum des Femurs eingetrieben. Liegt eine sub- oder intratrochantäre Fraktur vor, wird zusätzlich die erfindungsgemässe Hülse 10 auf das proximale Nagelende 3 aufgeschoben.

Die Hülse 10 besteht im wesentlichen aus einem gegen unten offenen Hohlzylinderabschnitt mit der Längsachse 11. Das proximale Ende 15 der Hülse 10 ist, abgesehen von der zentralen Bohrung 16, geschlossenen, so dass beim Aufschieben der Hülse 10 auf den Marknagel 1 gegenüber diesem eine axiale Anschlagsposition erreicht wird. Auf entgegengesetzten Seiten des Zylindermantels 14 der Hülse 10 sind zwei Öffnungen 12,13 angebracht, die beim Aufschieben der Hülse 10 und Erreichen der Anschlagsposition am proximalen Ende 3 des Marknagels 1 in den Bereich dessen Langlochs 5 positioniert werden. Dadurch entsteht ein den Marknagel 1 (durch das Langloch 5) und die Hülse 10 (durch die Öffnungen 12,13) durchquerender Kanal 12,5,13 zur Aufnahme eines Knochenfixationsmittel 20, der in den Fig. 7 und 8 als Knochenschraube realisiert ist.

Je nach Art und Lage der im Zylindermantel 14 der Hülse 10 angebrachten Öffnungen 12,13 können verschiedenartige und verschieden dimensionierte Knochenfixationsmitteln 20 durch die auf den proximalen Abschnitt 6 des Marknagels 1 geschobene Hülse 10 und durch das Langloch 5 des Marknagels 1 eingebracht werden. Neben den in den Fig. 7 und 8 dargestellten Knochenschrauben, eignen sich hierzu vorzugsweise sogenannte Knochenklingen, welche im Detail in der EP-A1 0 491 138 beschrieben sind und insbesondere zur Versorgung von Knochenbrüchen in Gelenksnähe, speziell im Bereich der proximalen und distalen Femurfrakturen, sowie an der proximalen Tibia. Um die Verwendung einer solchen Knochenklinge zu ermöglichen, sind die Öffnungen 12,13 schlitzförmig ausgebildet.

Nach erfolgter Hindurchführung eines Knochenfixationsmittel 20 durch die Öffnungen 12,13 und das dazwischenliegende Langloch 5 sind Hülse 10 und Marknagel 1 im wesentlichen axial gegeneinander fixiert. Die axiale Verschiebbarkeit der Hülse 10 gegenüber dem Marknagel 1 sollte höchstens 20 %, vorzugsweise höchstens 10% der Gesamtlänge des Langlochs 5 betragen. Bei einer beispielhaften Länge des Langlochs von 25 mm sollte die Verschiebbarkeit somit unter 5 mm, vorzugsweise unter 2,5 mm liegen. Bevorzugt wird jedoch eine axiale Verschiebbarkeit von höchstens 5 % und vorzugsweise von höchstens 2 %.

Das Knochenfixationsmittel 20 kann innerhalb eines weiten Winkelbereiches gegenüber der Längsachse 4 geneigt sein. Die Verbindungslinie durch die Zentren der korrespondierenden Öffnungen 12,13 können einen Winkel von 30° - 150°, vorzugsweise von 45° - 135° mit der Längsachse 4 einschliessen. Vorzugsweise wird das Knochenfixationsmittel 20 jedoch nicht unter einem 90°-Winkel eingeführt, d.h. mit anderen Worten, dass die Öffnungen 12 und 13 vorzugsweise nicht auf der gleichen Höhe angebracht sind. Besonders vorteilhaft ist es, wenn die eine Öffnung 13 zum proximalen Ende des Langlochs 5 und die andere Öffnung 12 zum distalen Ende des Langlochs 5 zu liegen kommen, wie dies in den Fig. 7 und 8 dargestellt ist. Bei einer solchen Anordnung reduziert sich die obengenannte Verschiebbarkeit von Hülse 10 und Marknagel 1 nach erfolgter Einführung des Knochenfixationsmittel 20 praktisch auf Null.

Die in der Hülse 10 proximal und quer zu dessen Längsachse 11 angebrachte Nute 22 besitzt die gleiche Dimension wie diejenige am proximalen Ende 3 des Marknagels 1. Die Nutensteine allfällig zu verwendender (zeichnerisch nicht dargestellter) Ein- und Ausschlag-Instrumente werden zweckmässigerweise so ausgelegt, dass sie durch ihr Eingreifen in die Nute 22 der Hülse 10 und in die Nute 21 des Nagels 1, diese beiden Elemente 1,10 zueinander positioniert und rotativ blockiert. Mittels einer (zeichnerisch nicht dargestellten) zentralen Schraube kann ein allfällig verwendetes Ein- und Ausschlag-Instrumentarium durch die Bohrung 16 der Hülse 10 in die Bohrung 17 mit Innengewinde 19 des Marknagels 1 eingeschraubt und fixiert werden. Diese Verschraubung fixiert dabei gleichzeitig die Hülse 10 am proximalen Nagelende 3. Mit dem fest aufgeschraubten Instrumentarium kann der Marknagel 1 in den Markraum eingetrieben werden. Je nach Ausführung der Öffnungen 12,13 der Hülse 10 kann ein entsprechendes Zielgerät auf das Einschlaginstrumentarium aufgesetzt werden. Durch dieses Zielgerät können die proximalen Knochenfixationselemente 20 gezielt durch die Öffnungen 12,13 und durch das Langloch 5 des Marknagels 1 eingesetzt werden.

Die erfindungsgemässe Kombination von Marknagel 1 und Hülse 10 vereinfacht die Adaptation des Implantats und die konkret zu versorgende Fraktur. Für den Kliniker bedeutet dies, dass er mit einem einzigen Marknageltyp sämtliche Femurschaftfrakturen und die verschiedenartigsten proximalen Femurfrakturen versorgen kann. Das einzige, was er pro Spezialindikation (d.h. in ca. 10 % der Fälle) zusätzlich braucht ist eine der Fraktur entsprechende Hülse 10. Diese Hülsen 10 sind nicht abhängig von der Länge oder dem Durchmesser des Marknagels 1. Ein Sortiment von beispielsweise fünf verschiedenen erfindungsgemässen Hülsen 10 kann somit ein Sortiment von etwa 200 verschiedenen, konventionellen Marknägeln ersetzen (5 verschiedene Marknageltypen mit ca. 4 verschiedenen Durchmessern und ca. 10 verschiedenen Längen). Diese Zahl der konventionellen Implantate ist in der Praxis noch um einiges höher, da es bei einzelnen Marknagelsystemen eine linke und eine rechte Ausführungsform gibt. Diese Implantateverdoppelung ist beim erfindungsgemässen System nicht nötig, da einfach die Hülse 10 um 180° gedreht werden kann, wie in den Fig. 7 und 8 dargestellt.
Eine ähnliche Reduktion gilt für die Instrumente, da ausser den verschiedenen Zielaufsätzen keine indikationsspezifische Instrumente für das erfindungsgemässe System benötigt werden.

Die auf den Marknagel 1 geschobene Hülse 10 wird durch das Knochenfixationselement 20 selbst befestigt, d.h. das Knochenfixationselement 20 sichert die Hülse 10 bezüglich Rotation und axialer Verschiebung nach distal. Eine mögliche axiale Verschiebung der Hülse 10 nach proximal kann durch eine Verschraubung der Hülse 10 mit dem Nagel verhindert werden. Dabei wird eine (zeichnerisch nicht dargestellte) Schraube durch die am proximalen Ende 15 der Hülse 10 angebrachte Bohrung 16 in das Innengewinde 19 der am proximalen Nagelende 3 vorhandenen Bohrung 17 eingebracht und festgedreht. Diese Sicherung ist jedoch nicht unbedingt erforderlich, da der Femur ausschliesslich unter axialen Druckkräften steht.
Muss das Knochenfixationselement 20 gegen Verschiebung längs dessen Achse 23 oder gegen Rotation fixiert werden, kann dies wiederum durch Ein- und Festdrehen einer (zeichnerisch nicht dargestellten) Schraube durch die am proximalen Ende 15 der Hülse 10 angebrachte Bohrung 16 in die am proximalen Nagelende 3 vorhandene Bohrung 17 mit Innengewinde 19 bis auf das Knochenfixationselement 20 hinunter erfolgen.

Als Materialien für den erfindungsgemässen Marknagel haben sich die in der Osteosynthese üblichen Implantatmaterialien bewährt.

## Patentansprüche

1. Marknagel mit einem distalen Ende (2), einem proximalen Ende (3), einer Längsachse (4) und einem im Bereich des proximalen Endes (3) angebrachten, quer zur Längsachse (4) verlaufenden Langloch (5) sowie eine auf das proximale Ende (3) des Marknagels (1) aufschiebbare, zylindrische Hülse (10), welche eine Zylinderachse (11), sowie mindestens zwei, auf entgegengesetzten Seiten des Zylindermantels (14) angebrachte Öffnungen (12,13) aufweist, wobei
A) die Hülse (10) derart ausgebildet ist, dass sie beim Aufschieben auf den Marknagel (1) gegenüber diesem in eine axiale Anschlagsposition bringbar ist; und
B) die Öffnungen (12,13) in der Anschlagsposition der Hülse (10) im Bereich des Langlochs (5) positioniert sind und mit letzterem einen, den Marknagel (1) und die Hülse (10) durchquerenden, Kanal (12,5,13) zur Aufnahme von Knochenfixationsmitteln (20) bilden,
**dadurch gekennzeichnet, dass**
C) die Öffnungen (12,13) schlitzförmig zur Aufnahme einer Knochenklinge ausgebildet sind.

2. Marknagel nach Anspruch 1, dadurch gekennzeichnet, dass die Hülse (10) im Bereich ihres, vorzugsweise geschlossenen, proximalen Endes (15) am proximalen Nagelende (3) in die axiale Anschlagsposition bringbar ist.

3. Marknagel nach Anspruch 1 oder 2, dadurch gekennzeichnet, dass die Öffnungen (12,13) derart positioniert sind, dass nach erfolgter Aufnahme der Knochenfixationsmittel (20) im Kanal (12,5,13) die axiale Verschiebbarkeit der Hülse (10) gegenüber dem Marknagel (1) höchstens 20 %, vorzugsweise höchstens 10% der Gesamtlänge des Langlochs (5) beträgt.

4. Marknagel nach Anspruch 3, dadurch gekennzeichnet, dass die axiale Verschiebbarkeit der Hülse (10) gegenüber dem Marknagel (1) höchstens 5 %, vorzugsweise höchstens 2 % der Gesamtlänge des Langlochs (5) beträgt.

5. Marknagel nach einem der Ansprüche 1 - 4, dadurch gekennzeichnet, dass die Öffnungen (12,13) nicht auf der gleichen Höhe liegen.

6. Marknagel nach Anspruch 5, dadurch gekennzeichnet, dass die Öffnungen (12,13) derart angeordnet sind, dass ein darin eingeführtes Knochenfixationsmittel (20) einen Winkel von 30° - 150°, vorzugsweise von 45° - 135° mit der Längsachse (4) einschliessen kann.

7. Marknagel nach einem der Ansprüche 1 - 6, dadurch gekennzeichnet, dass die zylindrische Hülse (10) an ihrem proximalen Ende (15), welches geschlossen ist, eine zentrale Bohrung (16) aufweist.

8. Marknagel nach einem der Ansprüche 1 - 7, dadurch gekennzeichnet, dass der Marknagel (1) an seinem proximalen Ende (3) eine zentrale Bohrung (17), vorzugsweise mit einem Innengewinde (19) aufweist.

9. Marknagel nach einem der Ansprüche 1 - 8, dadurch gekennzeichnet, dass der Marknagel (1) an seinem proximalen Ende (3) eine quer zur Längsachse (4) verlaufende Nute (21) aufweist.

10. Marknagel nach Anspruch 9, dadurch gekennzeichnet, dass die Hülse (10) an ihrem proximalen Ende (15) eine mit der Nute (21) des Marknagels (1) korrespondierende Nute (22) aufweist.

11. Marknagel nach einem der Ansprüche 1 - 10, dadurch gekennzeichnet, dass Marknagel (1) und Hülse (10) gegeneinander drehbar ausgebildet sind.

12. Marknagel nach einem der Ansprüche 1 - 11, dadurch gekennzeichnet, dass die schlitzförmigen Öffnungen (12,13) die Form eines Langloches aufweisen.

## Claims

1. Marrow nail with a distal end (2), a proximal end (3), a longitudinal axis (4) and a slot (5) in the area of the proximal end (3) running transverse to the longitudinal axis (4) as well as a cylindrical casing (10) which can be slid onto the proximal end (3) and exhibits a cylindrical axis (11) as well as at least two openings (12;13) placed at opposite ends of the cylindrical cover (14), whereby
A) the casing (10) is formed in such a way that upon being slid onto the marrow nail (1) it can be brought into an axial fastening position relative to the nail (1); and
B) the openings (12;13) are thereby positioned in the fastening position of the casing (10) in the area of the slot (5) and form with the latter a channel (12;5;13) running transversely through the marrow nail (1) and the casing (10) to admit bone fixation devices (20),
characterized in that
C) the openings (12;13) are in the form of slots for receiving a bone blade.

2. Marrow nail according to claim 1, characterized in that the casing (10) is transportable in the axial fastening position in the area of its preferably closed proximal end (15) at the proximal end (3) of the nail (1).

3. Marrow nail according to claim 1 or 2, characterized in that the openings (12;13) are so positioned that when a bone fixation device (20) is inserted into the transverse channel (12;5;13) the axial displacement of the casing (10) with respect to the marrow nail (1) is at most 20 %, preferably at most 10 % of the total length of the slot (5).

4. Marrow nail according to claim 3, characterized in that the axial displacement of the casing (10) with respect to the marrow nail (1) is at most 5 %, preferably at most 2 % of the total length of the slot (5).

5. Marrow nail according to one of the claims 1 - 4, characterized in that the openings (12;13) don't lie at the same height.

6. Marrow nail according to claim 5, characterized in that the openings (12;13) are placed such that a bone fixation device inserted therein may form an angle of 30° - 150°, preferably of 45° - 135° with the longitudinal axis (4).

7. Marrow nail according to one of the claims 1 - 6, characterized in that the cylindrical casing (10) at its proximal end (15) which is closed has a central bore hole (16).

8. Marrow nail according to one of the claims 1 - 7, characterized in that the marrow nail (1) at its proximal end (3) has a central bore hole (17), preferably with an internal thread (19).

9. Marrow nail according to one of the claims 1 - 8, characterized in that the marrow nails (1) at its proximal end (3) has a groove (21) transverse to its longitudinal axis (4).

10. Marrow nail according to claim 9, characterized in that the casing (10) at its proximal end (15) has a groove (22) that corresponds with the groove (21) of the marrow nail (1).

11. Marrow nail according to one of the claims 1 - 10, characterized in that the marrow nail (1) and the casing (10) are configured to be able to be turned against each other.

12. Marrow nail according to one of the claims 1 - 11, characterized in that the slotted openings (12;13) have the form of an elongated hole.

## Revendications

1. Clou médullaire comportant une extrémité distale (2), une extrémité proximale (3), un axe longitudinal (4) et un trou oblong (5) prévu dans la région de l'extrémité proximale (3) et s'étendant transversalement par rapport à l'axe longitudinal (4), ainsi qu'une douille cylindrique (10), pouvant être glissée sur l'extrémité proximale (3) du cou médullaire (1), qui présente un axe de cylindre (11) ainsi qu'au moins deux ouvertures (12, 13) prévues sur les côtés opposés de l'enveloppe cylindrique (14), tandis que
A) lors de son glissement sur le cou médullaire (1), la douille (10) est configurée de manière à pouvoir être amenée dans une position de butée axiale; et
B) lorsque la douille (10) est en position de butée, les ouvertures (12, 13) sont positionnées dans la région du trou oblong (5) et forment avec ce trou oblong un canal (12, 5, 13) de réception de moyens de fixation de l'os (20) qui traverse le cou médullaire (1) et la douille (10),
caractérisé en ce que
C) les ouvertures (12, 13) sont réalisées en forme de fente en vue de la réception d'une lame pour os.

2. Clou médullaire selon la revendication 1, caractérisé en ce que la région de l'extrémité proximale (15), de préférence fermée, de la douille (10) peut être amenée dans la position de butée axiale prévue à l'extrémité proximale (3) du clou.

3. Clou médullaire selon la revendication 1 ou 2, caractérisé en ce que lorsque les ouvertures (12, 13) sont positionnées de telle sorte que les moyens de fixation de l'os (20) ont été reçus dans le canal (12, 5, 13), la possibilité de coulissement axial de la douille (10) par rapport au clou médullaire (1) vaut au plus 20 %, et de préférence au plus 10 % de la longueur totale du clou médullaire (5).

4. Clou médullaire selon la revendication 3, caractérisé en ce que la possibilité de coulissement axial de la douille (10) par rapport au clou médullaire (1) vaut au plus 5 % et de préférence au plus 2 % de la longueur totale du trou oblong (5).

5. Clou médullaire selon l'une des revendications 1 à 4 caractérisé en ce que les ouvertures (12, 13) ne sont pas situées à même hauteur.

6. Clou médullaire selon la revendication 5, caractérisé en ce que les ouvertures (12, 13) sont disposées de manière à ce qu'un moyen de fixation de l'os (20) qui y a été inséré puisse former avec l'axe longitudinal (4) un angle compris entre 30° et 150°, et de préférence entre 45° et 135°.

7. Clou médullaire selon l'une des revendications 1 à 6, caractérisé en ce que la douille cylindrique (10) présente un alésage central (16) à son extrémité proximale (15), qui est fermée.

8. Clou médullaire selon l'une des revendications 1 à 7, caractérisé en ce que le clou médullaire (1) présente à son extrémité proximale (3) un alésage central (17) qui est de préférence doté d'un filet interne (19).

9. Clou médullaire selon l'une des revendications 1 à 8, caractérisé en ce que le clou médullaire (1) présente à son extrémité proximale (3) une rainure (21) qui s'étend transversalement par rapport à son axe longtudinal (4).

10. Clou médullaire selon la revendication 9, caractérisé en ce que la douille (10) présente à son extrémité proximale (15) une rainure (22) qui correspond à la rainure (21) du clou médullaire (1).

11. Clou médullaire selon l'une des revendications 1 à 10 caractérisé en ce que le clou médullaire (1) et la douille (10) sont configurés de manière à pourvoir tourner l'un par rapport à l'autre.

12. Clou médullaire selon l'une des revendications 1 à 11, caractérisé en ce que les ouvertures (12, 13) en forme de fente présentent la forme de trous oblongs.
